Europäisches Patentamt

European Patent Office

Office européen des brevets

(11) Veröffentlichungsnummer: **0 549 964 A2**

## (12) EUROPÄISCHE PATENTANMELDUNG

(21) Anmeldenummer: **92121437.5**

(22) Anmeldetag: **17.12.92**

(51) Int. Cl.5: **C12N 9/64**, //A61K37/465

(30) Priorität: **24.12.91 DE 4142908**

(43) Veröffentlichungstag der Anmeldung:
**07.07.93 Patentblatt 93/27**

(84) Benannte Vertragsstaaten:
**AT BE CH DE DK ES FR GB GR IE IT LI LU MC NL PT SE**

(71) Anmelder: **OCTAPHARMA AG**
**Schweizerhofstrasse 1**
**CH-8750 Glarus(CH)**

(72) Erfinder: **Bal, Frederic**
**Joseph-Städter-Strasse 74 Tür 7**
**A-1080 Wien(AT)**
Erfinder: **Sachse, Hans, Dr.**
**Schönburgstrasse 24/33**
**A-1100 Wien(AT)**

(74) Vertreter: **Meyers, Hans-Wilhelm, Dr. et al**
**Patentanwälte, von Kreisler Selting Werner,**
**Deichmannhaus am Hauptbahnhof**
**W-5000 Köln 1 (DE)**

(54) **Verfahren zur Herstellung eines virusinaktivierten Prothrombin-komplex-Konzentrats (PPSB).**

(57) Es wird ein Verfahren beschrieben zur Herstellung eines virusinaktivierten Prothrombinkomplex-Konzentrats (PPSB-Präparat) mit Prothrombin (Faktor II), Proconvertin (Faktor VII), Stuart-Prower-Faktor (Faktor X) sowie Hämophilie-B-Faktor (Faktor IX), wobei stabilisiertes Plasma an Ionenaustauschermaterialien chromatographiert wird, dadurch gekennzeichnet daß,
a) citratstabilisiertes Plasma mit einem Anionenaustauschermaterial auf Basis von QAE modifiziertem Dextran behandelt wird,
b) nach der Elution
c) mit einem geeigneten Puffer einer ersten Ultrafiltration unterzogen wird,
d) den so erhaltenen PPSB-Rohextrakt an DEAE modifiziertem Dextran chromatographiert wird,
e) danach mit einem geeigneten Puffer eluiert wird.

Die Erfindung betrifft ein Verfahren zur Herstellung eines virusinaktivierten Prothrombinkomplex-KonzentratS (PPSB-Präparat) gemäß Oberbegriff des Patentanspruchs 1 sowie Blutplasmafraktionen erhältlich gemäß diesem Verfahren.

PPSB ist ein Mischpräparat aus vier unterschiedlichen Gerinnungsfaktoren, die bei einer Vielzahl von medizinischen Indikationen verabreicht werden. Das Anwendungsgebiet erstreckt sich nicht nur auf Hämophiliepatienten, sondern auch auf chirurgisch zu versorgende Unfallopfer, Intensivpatienten, Gebärende oder sogar Patienten, die vor einer Zahnbehandlung stehen, bei der starke Blutverluste zu befürchten sind. PPSB besteht aus den Faktoren Prothrombin (Faktor II), Prokonvertin (Faktor VII), Stuart-Prower-Faktor (Faktor X) sowie Hämophilie-B-Faktor (Faktor IX).

In herkömmlicher Weise wird der Prothrombinkomplex PPSB aus bestimmten Plasmafraktionen, die mit EDTA versetzt worden sind, durch Adsorption an Kalziumphosphat gewonnen. Das Kalziumionen komplexierende Mittel EDTA (Ethylendiamintetraessigsäure) vernichtet jedoch die Faktoren VIII und V. Im Endeffekt werden die zur Herstellung des Prothrombinkomplex-Konzentrats bestimmten Plasmaproben somit für die Gewinnung der genannten wichtigen anderen Faktoren unbrauchbar gemacht.

Eine andere Methode besteht darin, PPSB an Ionenaustauschermaterialien zu binden aus mit Zitrat versetzten Plasmaproben. Nach den im Stand der Technik verwendeten chromatographischen Methoden werden im wesentlichen jedoch nur Proteinkomplexkonzentrate gewonnen, die den Faktor VII nicht oder nur unzureichend enthalten, so daß dieser später, durch andere Methoden gereinigt, wieder hinzugegeben werden muß. Dieses Verfahren ist umständlich und teuer, da im Grunde genommen zwei Präparationen durchgeführt werden müssen mit entsprechendem Aufwand an Ausgangsmaterial.

Die EP 0 131 740 A2 beschreibt ein Verfahren zur Herstellung nicht denaturierter, virusfreier, biologisch aktiver Proteinderivate aus Vollblut, Plasma, Serum, Plasmakonzentrat, Kryopräzipitat und andere aus Vollblut erhältlichen Fraktionen. Die Virusinaktivierung auch von Prothrombinkomplex-Faktoren (Faktor II, VII, IX und X) kann mit Hilfe der dort beschriebenen Verfahren durchgeführt werden.

Das der Erfindung zugrundeliegende technische Problem besteht darin, ein Verfahren anzugeben, mit dem ein Prothrombinkomplex-Konzentrat (PPSB) gewonnen werden kann, wobei das als Rohmaterial verwendete Blutplasma intensiver als bisher genutzt werden kann, so daß neben dem Prothrombinkomplex-Konzentrat auch weitere wertvolle Proteinbestandteile, beispielsweise Faktor VIII oder Faktor V, nach der Gewinnung des PPBS aus dem selben Rohstoff hergestellt werden können. Desweiteren soll das Verfahren alle vier essentiellen Bestandteile des Prothrombinkomplex-Konzentrates isolierbar machen, ohne daß nachträglich Komponenten hinzugefügt werden müssen.

Das der Erfindung zugrundeliegende Problem wird gelöst durch ein Verfahren mit den Merkmalen des Anspruchs 1. Die Unteransprüche betreffen bevorzugte Ausführungsformen des erfindungsgemäßen Verfahrens. Die Ansprüche 16 und 17 betreffen Blutplasmafraktionen, die erhältlich sind durch das erfindungsgemäße Verfahren.

Ausgehend von zitratstabilisiertem Plasma wird bei dem erfindungsgemäßen Verfahren in einem Schritt a) Anionenaustauschermaterial auf Basis von mit QAE (Quaternary Ammoniumethyl)Gruppen modifiziertem Dextran, eingesetzt.

Das stabilisierte Plasma wird in an sich bekannter Weise entweder in einem Batch-Verfahren mit dem Anionenaustauschermaterial in Kontakt gebracht oder aber im Durchflußverfahren an dem Material, welches in einer Säule befindlich ist, adsorbiert. Es ist vorteilhaft, dem Schritt a) eine Kryopräzipitation vorzuschalten und das sogenannte kryoarme Plasma im erfindungsgemäßen Verfahren einzusetzen.

Es hat sich als vorteilhaft erwiesen, als Anionenaustauscher QAE modifizierte Sephadex[R], ein Dextranderivat der Firma Pharmacia zu verwenden, welches unter der Bezeichnung QAE-Sephadex A50 erhältlich ist. Dieses Material ist ein zu einem bestimmten Grade quervernetztes Dextran. Die Ausschlußgröße des Materials (Größe der Moleküle, die in der Matrix noch retardiert werden) beträgt 200 Kilo-Dalton.

Vorzugsweise wird das Plasma oder das kryoarme Plasma in einem Behälter mit dem Anionenaustauschermaterial auf Basis von QAE modifiziertem Dextran in der Stufe a) in Kontakt gebracht. Es können nach der Adsorption an dem Anionenaustauschermaterial ein oder mehrere Waschschritte durchgeführt werden. Der Puffer, der vorzugsweise verwendet wird, um den Prothrombinkomplex an dem Ionenaustauscher zu adsorbieren, enthält Natriumchlorid in einer Menge von 450 bis 550, vorzugsweise 500 g in 60 kg Wasser. Die Osmolalität soll zwischen 200 und 400 mosm, insbesondere ca. 300 mosm, betragen.

Die Waschpuffer, die gegebenenfalls im Batch-Verfahren oder der Säule in Kontakt gebracht werden, soll eine etwas höhere Natriumchloridkonzentration enthalten und zusätzlich eine Puffersubstanz, insbesondere Tris(hydroxymethyl)-aminomethan. Die Natriumchloridmenge soll vorzugsweise im Bereich von 500 bis 600, insbesondere 560 g Kochsalz in 48 kg Wasser betragen. Die Puffersubstanz soll in einer Menge zwischen 45 und 70 g pro 48 kg Wasser liegen. Der pH-Wert soll ca. 8,0 betragen. Die Osmolalität soll im Bereich von 350 bis 450 mosm liegen.

Im erfindungsgemäßen Verfahren werden zur Elution des an dem Anionenaustauschermaterial adsorbierten Prothrombinkomplexes vorzugsweise Puffer eingesetzt, mit Natriumchlorid und Trinatriumzitrat in Wasser. Eine bevorzugte Zusammenstellung enthält 900 bis 960 g Natriumchlorid sowie 30 bis 40 g Trinatriumzitrat in 8 kg Wasser bei einem pH von 6 bis 8. Die Osmolalität sollte zwischen 3.800 und 4.200 mosm, vorzugsweise ca. 4.000 mosm, betragen. Der zur Elution verwendete Puffer zeichnet sich mithin durch eine relativ hohe Ionenstärke im Vergleich zu den vorher verwendeten Puffern aus. Ist die Adsorption in einem Batch-Verfahren durchgeführt worden, empfiehlt es sich, das Ionenaustauschermaterial in einen zylindrischen Hohlkörper, vorzugsweise eine Säule, zu füllen und die Elution des PPSB-Konzentrats in dieser Säule durchzuführen. Diese Vorgehensweise hat den Vorteil, daß sich eine Fraktionierung leichter durchführen läßt.

Die Elution kann bei Raumtemperatur erfolgen, wohingegen die vorher durchgeführten Schritte vorzugsweise bei einer Temperatur von 4 bis 10 °C durchgeführt werden.

Nach der Elution werden die das PPSB enthaltende Roheluat mittels einer Ultrafiltration konzentriert. Die Ultrafiltration erfolgt gegen einen Puffer, der sich durch eine geringe Natriumsalzkonzentration auszeichnet. Vorzugsweise enthält der Puffer 70 bis 100 g Natriumchlorid sowie als Puffersubstanz vorzugsweise einen Phosphatpuffer, insbesondere Dinatriumhydrogenphosphat in Mengen von 20 bis 35 g jeweils in 30 kg Wasser. Der pH-Wert dieser Pufferlösung soll zwischen 5,5 und 6,5, vorzugsweise ca. 6,0, betragen. Die Osmolalität soll vorzugsweise zwischen 55 und 75 mosm, besonders bevorzugt ca. 65 mosm, betragen.

Das vorzugsweise durch Ultrafiltration erhaltene PPSB-Rohkonzentrat kann an dieser Stelle vor der Weiterverarbeitung zur Virusinaktivierung mit Detergenzien behandelt werden. Dabei wird vorzugsweise 4 bis 10 Stunden, insbesondere 6 Stunden, bei Raumtemperatur oder leicht erhöhter Temperatur inkubiert. Es hat sich als besonders bevorzugt eine Behandlung mit TNBP (Tri-N-Butylphosphat in Kombination mit Tween 80), einem nicht ionischen Detergenz, bewährt. Dieses Verfahren ist Gegenstand eines Patentes des New York Blood Centers (EP-B-0 131 740). Danach wird nach Entfernung des Detergenzes in an sich bekannter Weise das PPSB-Rohkonzentrat in einem Puffer aufgenommen, der 150 bis 200 g Dinatriumhydrogenphosphat in 200 kg Wasser enthält. Der pH-Wert dieses Puffers soll zwischen 5,5 und 6,5 liegen und insbesondere 6,0 ± 0,1 sein. Die Osmolalität dieses Puffers ist relativ gering und soll zwischen 10 und 20 mosm liegen. Aus diesem Puffer heraus wird eine Sepharose[R] DEAE-Säule mit dem Rohkonzentrat beschickt. Es erfolgt vorzugsweise ein weiterer Waschschritt mit einem Puffer, der zwischen 380 und 420, insbesondere 400 g, Natriumchlorid sowie 30 bis 40 g Dinatriumhydrogenphosphat in 40 kg Wasser enthält. Der pH-Wert dieses Puffers soll ebenfalls einen Bereich von 5,5 bis 6,5 nicht über- oder unterschreiten. Insbesondere sollte der pH-Wert im Bereich vom 6,0 liegen. Die Osmolalität beträgt zwischen 290 und 340 mosm, vorzugsweise ca. 315 mosm.

Die Elution des an der Säule adsorbierten Materials erfolgt dann mit einem Puffer, der wiederum eine höhere Ionenstärke aufweist, nämlich beispielsweise 820 bis 900 g Natriumchlorid sowie 30 bis 40 g Dinatriumhydrogenphosphat in 40 kg Wasser bei einem pH-Wert von 5,5 bis 6,5, vorzugsweise ca. 6,0. Die Osmolalität soll im Bereich von 700 bis 800 mosm, vorzugsweise ca. 750 mosm, liegen.

Das mit dem Elutionsschritt gewonnene Präparat ist ein PPSB-Reineluat, welches durch eine Ultrafiltration gegen einen Puffer geringerer Ionenstärke konzentriert wird bis auf etwa 30 IU/ml des Faktor IX-Gehaltes [kalibriert gegen den 2nd International Standard, 87/532 (WHO-Standard)]. Der bei der Ultrafiltration verwendete Puffer enthält vorzugsweise Trinatriumzitrat in einer Menge von 300 bis 400 g in 60 kg Wasser und ist auf einen pH von 6 bis 8, vorzugsweise neutral, eingestellt. Die Osmolalität soll 50 bis 70 mosm, besonders bevorzugt ca. 60 mosm, betragen. Bei der Messung der Aktivität des PPSB-Verfahrens wird gemäß dem zitierten Standard Faktor IX als Leitparameter gemessen.

Das nach der Ultrafiltration gewonnene Präparat kann vorzugsweise sterilfiltriert und danach lyophilisiert werden. Das Präparat ist in dieser Form bereits handelsfähig.

Daher werden erfindungsgemäß auch Blutplasmafraktionen, die nach dem vorstehend genannten Verfahren gewonnen wurden, beansprucht.

Das erfindungsgemäße Verfahren wird anhand des nachfolgenden Beispiels näher erläutert.


Puffersysteme


Puffer 1: Natriumchlorid                                    0,15 mol/l

          Osmolalität                                       300 mosm

```
Puffer 2:  Natriumchlorid                          0,2   mol/l
           Tris(hydroxymethyl)-aminomethan    0,01 mol/l
           pH-Wert-Einstellung mit 1,0 M HCl
           auf 8,0 ± 0,1;
           Osmolalität ca.                         400 mosm


Puffer 3:  Natriumchlorid                          2,0   mol/l
           Trinatriumzitrat.2H₂O                0,015 mol/l
           pH-Wert-Einstellung auf 7,0 ± 1
           Osmolalität                    ca. 4.000 mosm


Puffer 4:  Natriumchlorid                         0,05   mol/l
           Dinatriumhydrogenphosphat.2H₂O    0,005 mol/l
           pH-Wert wird eingestellt mit 0,1 M HCl bis
           6,0 ± 0,1
           Kontrolle Osmolalität          ca.    65 mosm


Puffer 5:  Dinatriumhydrogenphosphat.2H₂O    0,005 mol/l
           pH-Wert-Einstellung mit 0,1 N HCl bis pH 6,0
           bis ± 0,1
           Osmolalität                    ca.    15 mosm


Puffer 6:  Natriumchlorid                         0,17   mol/l
           Dinatriumhydrogenphosphat.2H₂O    0,005 mol/l
           pH-Wert-Einstellung erfolgt mit 1,0 N HCl auf
           6,0 ± 0,1
           Osmolalität                     315 ± 10 mosm


Puffer 7:  Natriumchlorid                         0,37   mol/l
           Dinatriumhydrogenphosphat.2H₂O    0,005 mol/l
           pH-Wert-Einstellung mit 1,0 N HCl
           bis pH 6,0 ± 1
           Osmolalität                    ca. 740 mosm


Puffer 8:  Trinatriumzitrat.2H₂O                  0,02   mol/l
           pH-Wert eingestellt mit 1 N HCl bis 7,0 ± 0,1
           Osmolalität                    ca.    60 mosm
```

300 kg Plasma werden einer Kryopräzipitation unterzogen. Das kryoarme Plasma, ca. 300 kg, werden in einem Batch mit QAE-Sephadex A50 im Puffer 1 bei 10°C unter Rühren für eine Zeit von 25 Minuten miteinander vermischt. Die Menge des Sephadex-Materials beträgt 600 g, die Menge an Puffer 1 etwa 60 l. Danach wird mit ca. 50 l des Puffers 2 gewaschen. Dies erfolgt in mehreren Schritten ebenfalls in einem Batch-Verfahren oder alternativ wird das Material in eine Säule entsprechender Kapazität gefüllt und dann im Durchlaufverfahren gewaschen.

Vor der Elution des an das QAE-Sephadex A50 Chromatographiematerial gebundenen Materials wird dieses Material in eine entsprechende Säule gefüllt und dann mit ca. 8 kg des Puffers 3 behandelt. Dabei eluiert das an die Säule gebundene PPSB enthaltende Material.

Das aus der Säule aufgefangene PPSB-Roheluat wird mittels Ultrafiltration gegen den Puffer 4 konzentriert. Der Puffer 4 wird in einer Menge von ca. 5 l angeboten. Das erhaltene PPSB-Konzentrat beträgt ungefähr 10 kg. Der Ultrafiltrationsschritt sowie die hierauf folgenden Schritte können bei Raumtemperatur durchgeführt werden.

Das gewonnene PPSB-Rohkonzentrat wird dann mittels einer Mischung von 0,3% TNBP/1,0% Tween 80 durch sechsstündige Inkubation bei 37 °C virusinaktiviert. Danach werden ca. 8 kg Sepharose-DEAE-FF-Säule-Austauschermaterial in einer Säule mit ca. 100 Liter Puffer 5 äquilibriert und das PPSB-Konzentrat aufgegeben (10 kg). Das Säulenmaterial wird mit etwa 40 l des Puffers 6 gewaschen, um danach mit ca. 25 l des Puffers 7 behandelt zu werden. Dabei wird das PPSB-Reineluat von der Säule freigesetzt. Die Fraktionen werden aufgefangen und gegen den Puffer 8 einer weiteren Ultrafiltration unterzogen. Die Menge des PPSB beträgt vorzugsweise 25 kg und die Menge des eingesetzten Puffers etwa 5 kg.

Die Konzentrierung mittels Ultrafiltration erfolgt solange, bis die Fraktion eine Aktivität des Faktors IX von 30 IU pro Milliliter aufweist. Das PPSB-Konzentrat beläuft sich schließlich als Reinkonzentrat auf ca. 3,5 kg. Nach einer Sterilfiltration wird das Präparat gefriergetrocknet und abgepackt.

**Patentansprüche**

1.  Verfahren zur Herstellung eines virusinaktivierten Prothrombinkomplex-Konzentrats (PPSB-Präparat) mit Prothrombin (Faktor II), Proconvertin (Faktor VII), Stuart-Prower-Faktor (Faktor X) sowie Hämophilie-B-Faktor (Faktor IX), wobei stabilisiertes Plasma an Ionenaustauschermaterialien chromatographiert wird, dadurch gekennzeichnet daß,
    a) citratstabilisiertes Plasma mit einem Anionenaustauschermaterial auf Basis von QAE modifiziertem Dextran behandelt wird,
    b) nach der Elution
    c) mit einem geeigneten Puffer einer ersten Ultrafiltration unterzogen wird,
    d) der so erhaltenen PPSB-Rohextrakt an DEAE modifiziertem Dextran chromatographiert wird,
    e) danach mit einem geeigneten Puffer eluiert wird.

2.  Verfahren nach Anspruch 1, wobei im Schritt a) eine Kryopräzipitation vorgeschaltet ist und das kryoarme Plasma eingesetzt wird.

3.  Verfahren nach Anspruch 1 und/oder 2, wobei als Anionenaustauscher QAE-Sephadex A50 verwendet wird.

4.  Verfahren nach mindestens einem der Ansprüche 1 bis 3, wobei die Stufe a) als Batch-Verfahren ausgebildet ist.

5.  Verfahren nach mindestens einem der Ansprüche 1 bis 4, wobei ein oder mehrere Waschschritte durchgeführt werden.

6.  Verfahren nach Anspruch 5, wobei als Waschpuffer ein Natriumchlorid-tris (hydroxymethyl) -aminomethan-Puffer mit einem pH-Wert von ca. 8,0 und einer Osmolalität von 400 mosm eingesetzt wird.

7.  Verfahren nach mindestens einem der Ansprüche 1 bis 6, wobei die Elution des in Stufe a) adsorbierten Materials mit einem Natriumchlorid Trinatriumcitrat-Puffer pH 6 bis 8 und einer Osmolalität von ca. 4.000 mosm durchgeführt wird.

8.  Verfahren nach Anspruch 7, wobei das Material, an welchem der Prothrombinkomplex adsorbiert ist, in eine Säule gefüllt wird und danach der Prothrombinkomplex eluiert wird.

9.  Verfahren nach mindestens einem der Ansprüche 1 bis 8, wobei die erste Ultrafiltration mit einem Natriumchlorid und Dinatriumhydrogenphosphat enthaltenden Puffer (pH 5 bis 7, Osmolalität ca. 65 mosm) durchgeführt wird.

10. Verfahren nach mindestens einem der Ansprüche 1 bis 9, wobei die Virusinaktivierung zwischen den Schritten c) und d) des Anspruchs 1 erfolgt.

11. Verfahren nach mindestens einem der Ansprüche 1 bis 10, wobei der Rohextrakt der Stufe d) des Anspruchs 1 an Sepharose DEAE chromatographiert wird mit einem Puffersystem enthaltend Dinatriumhydrogenphosphat bei einem pH-Wert von ca. 6,0 mit einer Osmolalität von ca. 15 mosm.

12. Verfahren nach mindestens einem der Ansprüche 1 bis 11, wobei nach Beladen der Säule gemäß Stufe d) die Säule mit einem Puffer enthaltend Natriumchlorid, Dinatriumhydrogenphosphat und einem pH-Wert von ca. 6,0 und einer Osmolalität von ca. 315 mosm gewaschen und das adsorbierte Material gemäß Stufe e) des Anspruchs 1 mit einem Puffer enthaltend Natriumchlorid und Dinatriumhydrogenphosphat mit einem pH-Wert von ca. 6,0 und einer Osmolalitat von ca. 750 mosm eluiert wird.

13. Verfahren nach mindestens einem der Ansprüche 1 bis 12, wobei der Elution der Stufe e) des Anspruchs 1 eine zweite Ultrafiltration als Stufe f) gegen einen Puffer enthaltend Trinatriumcitrat bei einem pH-Wert von ca. 7,0 und einer Osmolalität von ca. 60 mosm folgt.

14. Verfahren nach Anspruch 13, wobei eine Konzentration des Prothrombinkonzentrats bis zu ca. 30 IU/ml (Second International Standard 87/532) erfolgt, wobei Faktor IX als Leitparameter dient.

15. Verfahren nach Anspruch 13 und/oder 14, wobei der Stufe f) eine Sterilfiltration und/oder Lyophilisation folgt.

16. Blutplasmafraktion erhältlich gemäß einem Verfahren gemäß Ansprüchen 1 bis 15.

17. Blutplasmafraktion nach Anspruch 16 im wesentlichen enthaltend den PPSB-Faktor.